(19) Europäisches Patentamt / European Patent Office / Office européen des brevets

(11) **EP 3 932 295 A1**

(12) **EUROPEAN PATENT APPLICATION**

(43) Date of publication:
**05.01.2022 Bulletin 2022/01**

(51) Int Cl.:
**A61B 5/00** *(2006.01)* **A61B 5/0275** *(2006.01)*
**A61B 5/0285** *(2006.01)*

(21) Application number: **20183260.7**

(22) Date of filing: **30.06.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Universität Zürich**
**8006 Zürich (CH)**

(72) Inventors:
• **DEÁN-BEN, Xosé Luís**
**8057 Zürich (CH)**
• **RAZANSKY, Daniel**
**8046 Zürich (CH)**

(74) Representative: **Kleine, Hubertus et al**
**Loesenbeck - Specht - Dantz**
**Patent- und Rechtsanwälte**
**Am Zwinger 2**
**33602 Bielefeld (DE)**

(54) **OPTOACOUSTIC TOMOGRAPHY METHOD OF A LIVING ORGANISM, 2D- OR 3D OPOTACOUSTIC IMAGES, 2D- OR 3D BLOOD FLOW AND LIGHT FLUENCE IMAGES AND A PHOTOABSORBING AGENT FOR THE USE IN AN OPTOACOUSTIC METHOD**

(57)  An optoacoustic tomography method of a living organism, especially a human or an animal, characterized by the following steps;

I Providing a living organism

II Positioning of an optoacoustic imaging system at the region of interest of the living organism.

III Intravenous Injection of an exogenous photoabsorbing agent as an emulsion containing

liquid and/or solid particles with a maximum diameter lower than 10 $\mu$m and

wherein the particles comprise at least a photoabsorbing substance

IV Acquisition of at least one image after the injection of the photoabsorbing agent in step III;

V Determination of data derived from said image based on analyzing the signals of a plurality of said particles;

  Several uses of the method; an optoacoustic 2D or 3D-Image; an optoacoustic blood flow image; an optoacoustic light fluence image and a photoabsorbing agent for the use in an optoacoustic method.

Fig. 2

EP 3 932 295 A1

**Description**

**Technical Field**

The subject of the present invention is an optoacoustic tomography method of a living organism, uses of said method in different applications, as well as an optoacoustic 2D- or 3D-image, an image for measuring the blood flow, an image for measuring the light fluence and a photoabsorbing agent for the use in an optoacoustic method.
The optoacoustic methods, such as the optoacoustic tomography method presented herein, are based on the effect of the formation of sound waves as a result of absorption in a material sample, which can be a tissue or a living organism, such as a human or an animal. In particular, it aims at rendering a structural 2D and 3D image with high resolution as well as the measuring the blood flow and the light fluence distribution inside the imaged tissue. For obtaining this effect, the light intensity should vary, either periodically by modulating the excitation light signal or by delivering a pulsed light signal.

**Background Art**

Abnormal microcirculatory changes in arterioles, venules and capillaries are key functional indicators of important diseases such as cancer, diabetes, cardiovascular conditions or neurodegenerative disorders. *In vivo* visualization of these structures has traditionally been restricted to the shallow depths smaller than 1 to 2 mm reachable with high-resolution optical imaging methods.
Optoacoustic (photoacoustic) tomography has enabled breaking through this light diffusion barrier to map functional hemodynamic parameters such as oxygen saturation in deep-seated vessels, but frequency-dependent attenuation of ultrasound waves limits the resolution and prevents reaching the capillary level.
Imaging of individual capillaries might be achieved via localization of microbubbles flowing in blood with pulse-echo ultrasound. Optoacoustic localization of light-absorbing particles in mammalian tissues is however hampered by the strong background absorption of blood.

**Object of the invention**

With regard to the aforementioned discussion of the topic, one object of the current invention is to provide a new optoacoustic method as well as new contrast agents to make it possible to perform localization-based imaging by means of optoacoustic tomography.
In this way it is possible to provide a suitable method for optoacoustic tomography with superior resolution and/or contrast of the images.
A further object of the invention is to provide new applications for said optoacoustic tomography method.

**Disclosure of the invention**

The current invention overcomes the aforementioned objections resulting from said state-of-the-art, especially related to the background absorption of blood, with the subject-matter of claim 1.
An inventive optoacoustic tomography method of a living organism, especially a human or an animal, comprising the following steps;

I Providing a living organism
Said providing may comprise the step of anesthetizing the living organism, which could preferably be a mouse but can also be other animals. Since said technique might develop further, also the imaging of human organs might be possible by the inventive method.
II Positioning an optoacoustic imaging system around the region of interest of the living organism and preferably performing optoacoustic imaging.
Said optoacoustic imaging might comprise the provision of images with a single laser pulse. Said optoacoustic imaging preferably allows a preview to define the exact location.
An optoacoustic system may especially comprise one or more of an electromagnetic radiation source configured to irradiate the object with one or more pulses of electromagnetic radiation; and a detector unit configured to detect acoustic waves which are generated in an imaged region of the object upon irradiation with one or more pulses of electromagnetic radiation.
III Intravenous Injection of an exogenous photoabsorbing agent as an emulsion containing
liquid and/or solid particles with a maximum diameter below 10 $\mu$m and
wherein the particles comprise at least a photoabsorbing substance

Said absorbing substance might be arranged in the emulsion outside, especially around, said particles and/or inside said particles. This definition also includes coating of the particles. The particles may preferably be monodisperse but can also be polydisperse.

In a preferred embodiment at least a part of said absorbing substance is encapsulated in the particles. Said particles therefore preferably comprise an encapsulation material and said absorbing substance. Said particles are also considered in a preferred embodiment in the context of the current invention as microdroplets.

IV Acquisition of at least one image after the injection of the photoabsorbing agent in step III;

Said acquisition might be performed directly after the injection of the photoabsorbing agent. The acquisition preferably may require an activation, especially by light in the visible or near infrared spectral range, such as at least one laser pulse. The acquisition has a better performance if the frame rate is higher than 50 Hz. The acquired images might preferably be at least 40 images, more preferably between 50 and 300 images.

V Determination of data derived from said image based on analyzing the signals of a plurality of said particles.

Said analyzing might comprise localizing and/or evaluating the sequence of images for the characterization of fluctuations created by the particles. Preferably there are different ways of analyzing the fluctuations. Those may include calculating the standard deviation or reconstructing with constrains and adding up images.

Said method can be used for different preferred purposes. A first purpose is the use of the inventive method to generate 3D and/or 2D optoacoustic images.

A further purpose might be the use of the inventive method to generate optoacoustic images representing blood flow.

A third purpose is the generation of optoacoustic images of the light fluence inside the tissue, which can be derived by said inventive optoacoustic method.

With respect to existing optoacoustic imaging methods, the current method provides a better spatial resolution, with at least a five-fold gain. The current inventive method enables the measurement of blood flow and light fluence for the first time. The achievable depth for receiving signals for the current inventive method is at least in about the same range as the existing methods.

Optical microscopy and other types of microscopy may also provide high quality images with micron-scale resolution but are limited to very shallow depths (< 1 mm). On the other hand, conventional full body tomography imaging modalities, such as X-ray, CT, MRI PET, SPECT or diffuse optics, do not provide microscopic resolutions. Furthermore, a blood flow measurement is not possible with these methods. Ultrasound localization methods exist that are not based on the optoacoustic effect but they are slow because of the need for extensive beamforming and compounding. This may cause difficulties for example when attempting to attain a 3D localization image. The bidirectional propagation of ultrasound waves further hampers the applicability of ultrasound localization methods to image through the skull or other strongly acoustically mismatched tissues.

Further advantageous embodiments of the inventive method are subject matter of the depending claims.

In a preferred embodiment of the invention the particles of said exogenous photoabsorbing agent might be smaller than the blood cells of the organism in which said agent is injected.

In a preferred embodiment of the invention the relation of the absorption and the average radius of said particles can be taken into account. For example, for a typical resolution of 150 $\mu$m, this may be derived from the following parameters:

$$\text{Concentration of dye in the particle} \leftarrow C_p \geq 15000 \frac{\varepsilon_{Hb}}{\varepsilon_p} \frac{V_{RBC}}{V_p} C_{Hb}$$

Extinction coefficient hemoglobin — $\varepsilon_{Hb}$
Volume red blood cell — $V_{RBC}$
Concentration hemoglobin in RBCs — $C_{Hb}$
Extinction coefficient dye — $\varepsilon_p$
Volume particle — $V_p$

For a typical near-infrared dye with the same volume as red blood cells, the $c_p$ value could be: $C_p \geq 300$mM

Also other parameters may have an influence for said optoacoustic inventive method. The control of the frequency (bandwidth) of the ultrasonic sensors of the optoacoustic system can determine the effective resolution of the system. If for example the resolution is doubled, the factor 15000 in the aforementioned example is divided by 8. Yet, operation in a higher resolution regime may lead to other challenges such as excessive acoustic distortions for the high frequency components.

A further parameter which may have an influence is the wavelength range, the preferred range might preferably be the near-infrared window (650-1300nm). For visible wavelengths, the particles must have larger size because of higher absorption of blood, while the light penetration depth is also lower. This might have disadvantages since larger particles could be trapped (blocked) within the living organism.

The optoacoustic imaging system might be provided with a laser, an ultrasonic transducer and a control and/or evaluation unit, wherein said optoacoustic imaging system is provided to generate an image with a single laser pulse.

As mentioned above, the said method further comprises a reconstruction step that for instance provides a preview, especially a real-time preview, for determining the exact anatomical location for an injection of said photoabsorbing agent preferably prior to the injection.

As mentioned above the acquisition of said at least one image is initiated shortly after the injection, this means preferably within less than 10 Minutes, most preferably within less than 60 Seconds after step III.

In a preferred embodiment of the invention the particles comprise at least dichloromethane, polycaprolactone and/or silica, preferably in a concentration of at least 25% mass. The aforementioned materials might preferably include the aforementioned encapsulation material. While dichloromethane can be provided as liquid particles, polycaprolactone and silica core particles can be solid particles.

The absorbing substance may preferably comprise a dye or nanoparticles. The dye can be a cation heptamethine fluorescent dye, preferably IR780 dye in a concentration preferably higher than 50 mM. The nanoparticles can be carbon nanotubes. Said absorbing substance may also be a mixture of different ingredients. IR780 dye has an absorption peak at about 780 nm while carbon nanotubes may have a broad absorption spectrum.

As an emulsion said photoabsorbing agent can preferably comprise a continuous, preferably aqueous, phase and a disperse phase with said particles.

More preferably the volumetric fraction of said disperse phase to said continuous phase is less than 5 %, preferably between 2-4%.

The molar concentration of the photoabsorbing substance, especially the dye, in or at the particles, may preferably be at least 50 mM, more preferably between 100-400 mM.

The control and/or evaluation unit of said optoacoustic imaging system can be provided such that said laser pulse for optoacoustic imaging can be short pulsed with a duration of less than 10 ns.

The mass of the injected bolus, or in other words the emulsion, of photoabsorbing agent is at most 10 g/kg weight of said animal or human, preferably between 1-5 g/kg.

Step V may be based on localization of individual particles, preferably derived from a local point spread function of said particles.

The inventive method may comprise a blood flow measurement. Said measurement may be performed such that the method in step V comprises a sequence of images within a predetermined time frame, wherein the movement of at least one or more individual particles is determined from said images.

The achievable depth of an optoacoustic image derived with the inventive method can preferably be between 0.5 and 20 mm.

A preferred laser pulse repetition rate used within said method can preferably be at least 1 Hz, more preferably 50-150 Hz.

The wavelength of said laser pulse may be within a range between 650-1300 nm, more preferably between 680-900 nm.

The inventive method may be used for different purposes. Due to the superior resolution achievable with the current inventive method, even new applications can be derived, which are reported in the context of optoacoustic tomography for the first time.

Said inventive method can be used to generate 2D and 3D optoacoustic images, optoacoustic blood flow images, meaning blood flow images derived from optoacoustic data, and optoacoustic light fluence images, meaning light fluence derived from optoacoustic data. Likewise said method can be used to measure the blood flow and/or the light fluence inside a tissue.

Also part of the current invention is an optoacoustic 2D or 3D image and/or an optoacoustic blood flow image and/or an optoacoustic light fluence image, preferably derived by the inventive method, wherein said image displays arterioles, venules and capillaries with a resolution of at least 50 $\mu$m, preferably between 5-25 $\mu$m. Said optoacoustic blood flow image can be used for measuring velocities, preferably up to 100 mm/s. The optoacoustic light fluence image of a tissue can be measured preferably as a function of excitation wavelength of a laser of an optoacoustic imaging system.

An inventive photoabsorbing agent can be used in an optoacoustic method, especially in an inventive method as described above, wherein said agent is an emulsion contains liquid and/or solid particles with a maximum diameter lower than 10 $\mu$m and wherein the particles comprise at least an absorbing substance.

Said particles are sufficiently small to flow throughout the vascular network (including the smallest capillaries) without being arrested (e.g. blockage of blood or the like).

Said particles should preferably be at least 0.5 $\mu$m, more preferably at least 2 $\mu$m to generate a strong signal for the detection in the presence of blood.

A photoabsorbing substance can be defined as a substance that absorbs light so that it can generate an optoacoustic signal. A preferred range for the pressure level of the acoustic signal measured at the detector location is higher than 1 Pascal.

**Brief Description of the drawings**

One advantageous embodiments for inventive process and optoacoustic images are further explained in detail below together with drawings. Specific parts of the different embodiments can be understood as separate features that can also be realized in other embodiments of the invention. The combination of features described by the embodiment shall not be understood as a limitation for the invention

Fig. 1   a schematic view of the steps of an embodiment of the current invention;
Fig. 2   a comparison picture of the resolution of image recorded by localization optoacoustic tomography and by optoacoustic tomography performed by the method currently used in the state of the art.

**Mode for Carrying out the invention**

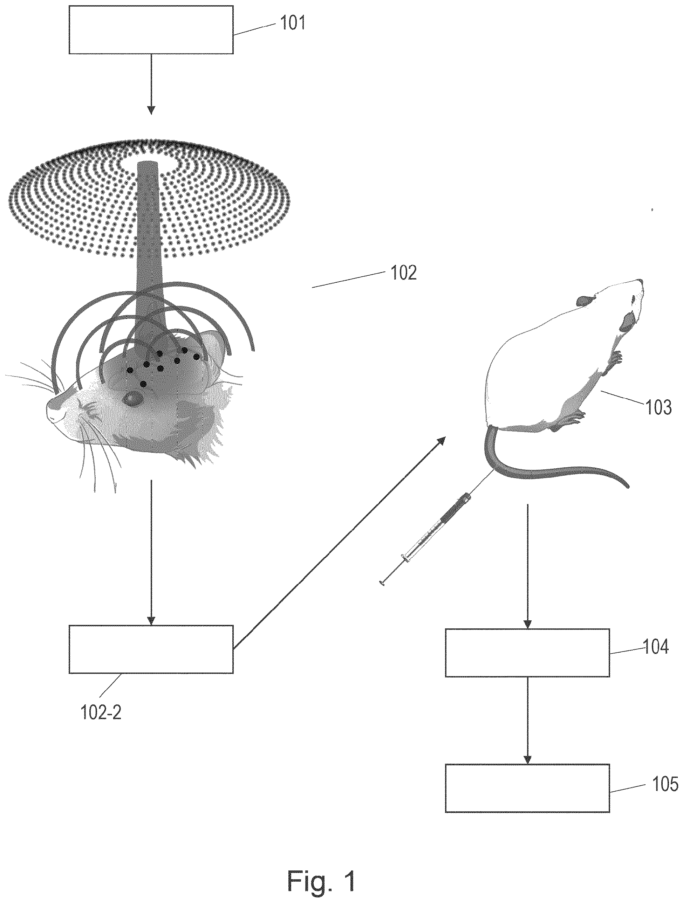

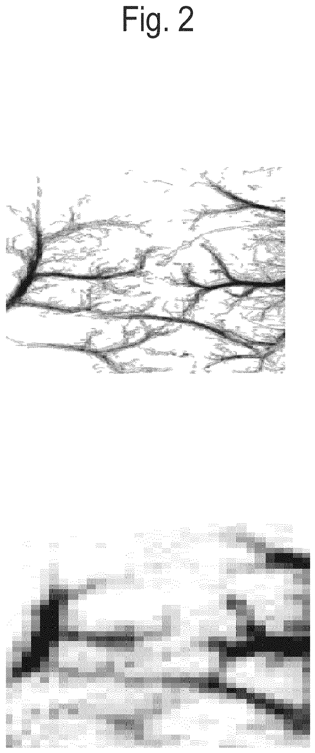

In a first step 101 of the inventive method a living organism can be provided. Said organism can preferably be a human or an animal. Said tomography can be performed on different organs of the organism. Preferably a tomography of the brain of said organism can be performed. The provided organism is still living, meaning that all steps in said inventive method can be performed *in vivo.* Said providing can comprise the anesthetization of said organism.
In one example the brain of a mouse has been analyzed by an optoacoustic imaging apparatus using said inventive method. Preferably, the providing of a living organism has been executed as described by the following experimental procedure:
Female mice (6-8 weeks old) are used for *in vivo* experiments. Mice are anaesthetized with isoflurane (3% v/v for induction and 1.5% during the experiments) in a mixture of air and $O_2$. Optoacoustic imaging of the brain region is performed with the head of the mouse being fixed into a custom-designed stereotactic mouse head holder coupled to a breathing mask through which anesthesia was provided. Blood oxygenation, heart rate and body temperature are continuously monitored, being the temperature maintained at ~36°C with a heating pad. Intravenous (IV) injection of 100 ml of a microdroplet emulsion of an inventive exogenous photoabsorbing agent is performed 30 s after the beginning of the acquisition, where the total acquisition time is 420 s. The mice are euthanized immediately after signal acquisition. One of the mice is maintained alive and fully recovers after the experiment. It is euthanized 2 days after injection of the microdroplets. No behavioral changes are observed in this period. Animal handling and all experiments are performed in full compliance with institutional guidelines.
Said optoacoustic imaging system can be positioned in a second step 102 at the region of interest of the living organism. Said optoacoustic imaging system can render optoacoustic images. This way background absorption of blood can be recorded and the biological material is activated to perform said optoacoustic effect. Preferred optoacoustic imaging system can be further described in detail in the following:
Optoacoustic imaging of the mouse brain is performed with a tomographic imaging system schematically illustrated in Fig. 1. Basically, a spherical array of 512 ultrasound sensing elements (Imasonic SaS, Voray, France) is used to collect the corresponding optoacoustic signals generated via excitation with an optical parametric oscillator (OPO)-based short-pulsed (<10 ns) laser (Innolas GmbH, Krailling, Germany) tuned to 780 nm, corresponding to the peak absorption of the exogenous photoabsorbing agent, which can preferably comprise a dye. The elements of the ultrasound array have 7 MHz central frequency and >80% detection bandwidth. The array features a central aperture with 8 mm diameter and 3 lateral apertures with 4 mm diameter located at 45° elevation angle and equally spaced (120°) in the azimuthal direction. Light is guided with a custom-made 4-arm fiber bundle (Ceram-Optec GmbH, Bonn, Germany) through the apertures of the array. This provided an approximately uniform illumination profile on the mouse brain surface with optical fluence <20 mJ/cm$^2$. The optoacoustic signals are digitized at 40 megasamples per second with a custom-made data acquisition system (DAQ, Falkenstein Mikrosysteme GmbH, Taufkirchen, Germany) triggered with the Q-switch output of the laser and transmitted to a computer via Ethernet.
Reconstruction of the optoacoustic images can be performed as a subsequent step 102-2, as further explained in detail within the following section:
Optoacoustic images for a volume of 10x10x5 mm$^3$ (100x100x50 voxels) can be reconstructed with a graphics processing unit (GPU)-based implementation of a back-projection formula. #
Said back-projection formula can be derived from the following literature: [Xu, M., & Wang, L. V. (2005). Universal back-projection algorithm for photoacoustic computed tomography. Physical Review E, 71(1), 016706.].
Prior to reconstruction, the collected signals are band-pass filtered with cut-off frequencies between 0.1 and 9 MHz. The optoacoustic images used for single droplet localization can be obtained by subtracting the background image taken before injection of the emulsion. This enables isolating the dynamically changing signal originating from the absorbing particles flowing in the vasculature from the static background signal coming from endogenous absorbers such as hemoglobin. The result can be seen in Fig. 2. Said reconstruction allows a preview to define the exact localization when the photoabsorbing agent has been injected.

In a third step 103, microdroplets with an average diameter of about 5.5 $\mu$m (+/- 0.5 $\mu$m) of an exogenous photoabsorbing agent are injected as an *in vivo* intravenous injection in a living organism, such as a mouse. The microdroplets have optical absorption which is higher than red blood cells for near-infrared wavelengths.

Said microdroplets can be produced by the following method:

A suspension of dichloromethane microdroplets in water is produced following a standard emulsification procedure. The disperse dichloromethane phase is prepared by diluting IR-780 iodide and dichloromethane to achieve a dye concentration of approximately 200 mM. The continuous (water) phase is prepared by adding 3% (v/v) Tween 20 surfactant in phosphate buffered saline. 50 $\mu$l of disperse phase and 1.5 ml of continuous phase are mixed in a 2 ml Eppendorf and vortexed at speed 9 during 30 s. The continuous phase is cooled down to approximately 4°C before vortexing to avoid vaporization of dichloromethane. The resulting emulsion is eventually filtered with a cell strainer with pore size 10 $\mu$m and subsequently inspected in a stereo microscope to check that no particles with irregular (non-spherical) shape arguably corresponding to the dye powder are present in the suspension.

In a fourth step 104 an acquisition of at least one image after the injection of the photoabsorbing agent is performed and in a fifth step 105 data is derived from said image based on analysing the signals of a plurality of said particles.

Said analysing and derivation could mean localization of said particles. Isolated microdroplets are strong absorbers smaller in size than the resolution of our imaging system. They should thus appear in the image as the local point spread function. In each reconstructed frame, local intensity maxima are thus detected, and small regions around these maxima are correlated to a model point spread function of an imaging system (the same empirical point spread function is used over the whole reconstructed volume). The maxima with correlation coefficients above 0.5 are considered as droplets. The localization of these droplets is then further refined using a local quadratic fitting of the intensity maximum, and their positions are stored.

Additionally, or alternatively, a sequence of images for analyzing the fluctuations created by the particles can be recorded. The said analysis may include calculating the standard deviation. A further option is the constrained reconstruction and adding up images. Also, the blood stream velocity can be measured by the aforementioned method.

A light fluence estimation can be performed in the following way:

The amplitude of the optoacoustic signal generated by a droplet is proportional to the amount of energy absorbed and the local flight fluence. The absorbed energy depends on the volume of the droplet, which changes according to the size distribution. However, the light fluence can be assumed to be proportional to the average intensity of the reconstructed optoacoustic signals for droplets at a given location. For fluence estimation, 50 droplets are selected in a cross-sectional image of the brain. The intensities of the differential optoacoustic images for the positions of the selected droplets are fitted to an exponentially decaying function. Specifically, a function of the form (a/z)exp(-bz) is used for least square fitting, being z=sqrt((x-x0)^2+(y-y0)^2)). Curve fitting results in optimum values of the parameters a, b, x0 and y0.

In one aspect of the current invention a localization optoacoustic tomography can be performed after said injection for rendering a three-dimensional imaging of said mouse, especially said mouse brain. Three-dimensional imaging of the mouse brain microvasculature through the intact scalp and skull is facilitated by the unidirectional propagation of ultrasound waves, yielding a resolution of ~20 $\mu$m.

The unique temporal resolution of state-of-the-art optoacoustic tomographic imaging may further enable accurately mapping the blood flow velocity within microvascular structures and the signal intensities of the localized particles could be used to estimate the light fluence distribution within optically diffuse tissues, a long-standing goal in biomedical optics.

Fig. 2 demonstrate the *in vivo* applicability of localization optoacoustic tomography by intravenous injection of extremely absorbing dichloromethane microdroplets with sizes in the order of the red blood cells. The small size of the droplets prevents capillary blockage, while their strong absorption facilitates individual detection and accurate localization within the field of view of the optoacoustic tomography imaging system with single shot excitation.

The basic principle of localization optoacoustic tomography along with a lay-out of the optoacoustic tomographic approach can be employed to image the mouse brain. A short-pulsed (nanosecond duration) laser tuned to an optical wavelength of 780 nm can be used to simultaneously illuminate the entire mouse brain and generate pressure (ultrasound) waves via thermal expansion that are eventually detected with a spherical array of transducers.

These pressure waves are produced by endogenous absorbers, mainly hemoglobin in red blood cells, as well as by the exogenously administered absorbing microdroplets required for localization optoacoustic tomography. This leads to a static background optoacoustic signal corresponding to vascular structures of different sizes superimposed to a dynamically changing signal generated at the particles flowing in blood. Localization optoacoustic tomography can only be performed if the signal generated by an individual particle is sufficiently high to be detected in an image voxel. The background signal per voxel is proportional to the number of red blood cells enclosed in it, which can reach hundreds to thousands. The dichloromethane droplets employed have an approximate concentration of 200 mM of IR-780 dye. The average size of the droplets can be 5.5 $\mu$m, as measured by a microscopic method, which is smaller than the disk diameter of red blood cells.

A background image along with an MRI image of the brain can serve as anatomical reference. The filtered optoacoustic image for a given time point can also be displayed. Microdroplets in arteries and in the cortical vasculature are clearly visible in the images derived by the inventive method. The flow of microdroplets can be visualized. A continuous flow of particles is observed over the entire imaging sequence, indicating that no capillary arrest is produced. This makes the photoabsorbing agent especially valuable for the medicinal use within an *in vivo* injection.

Specifically, the maximum intensity projections (MIPs) along the z and x direction can be displayed in an image. The trajectory of the droplet as it flows from a deep-seated artery to the superficial cortical vasculature can be detected and displayed as well. It is also possible for an optoacoustic tomography system to cover a 3D region with single shot excitation, which is essential for an accurate estimation of the trajectory, while the high temporal resolution is also crucial to accurately quantify the high speed of the blood flow.

Localization images can be built by aggregating the localized positions of the microdroplets flowing through the vascular network over a sequence of optoacoustic images. No average or compounding of optoacoustic frames is required for droplet localization. This is of advantage considering the high blood flow velocity in the mouse brain, i.e., the effective integration time of the images used for particle localization must be minimized. Optoacoustic images acquired with a single laser pulse (single shot excitation) are very well defined in time by the duration of the laser pulse and hence facilitate accurate localization of particles moving with a very high speed. The image formation process in localization optoacoustic tomography can be visualized in a movie.

The maximum intensity projection (MIP) of the volumetric (3D) images clearly reveals structures oriented in the vertical (axial) direction, such as arterioles penetrating the brain cortex. Such vertically oriented structures cannot be resolved in standard OA images not only because of the lower resolution but also as a result of the so-called limited-view effects arising from the limited angular tomographic view of the imaging system. In localization optoacoustic tomography, those could be followed over a length of ~1 mm, which matches the cortex thickness in this region. A 3D view corresponding to a zoom-in of one of these vertical structures can also be performed. Vascular structures separated by 22 $\mu$m could be resolved for a *depths of ~1.5 mm* through the intact scalp and skull. This serves as an estimate of the achievable resolution in this scenario. It is important to note that the spatial resolution in localization optoacoustic tomography depends on the number of droplets passing through the reconstructed voxel ($10 \times 10 \times 10 \mu m^3$) and thus could be potentially improved by using a longer acquisition time or a higher droplet concentration (approximately 20 droplets were localized in each volumetric frame). The high temporal resolution of the optoacoustic tomography imaging system employed further enables estimating the blood flow velocity via particle tracking. The capability to provide 3D images with single shot excitation is of high advantage for this purpose.

With said data a 3D view of the estimated velocity map can be derived. The obtained results - from several cm.s$^{-1}$ in larger vessels like the medium cerebral artery down to 5 mm.s$^{-1}$ in small vessels - are consistent with previously reported values for the mouse brain. It is further possible to assess the flow velocity profile within relatively large vessels. Droplet velocities aggregated in an example in 20$\mu$m bins over a 150$\mu$m vessel cross-section, reveals a Poiseuille flow profile.

The feasibility to distinguish velocity values in different bins with statistical significance is in good agreement with the 20$\mu$m resolution found in selected profiles of the localization optoacoustic tomography images. The capability to quantify the blood flow represents an important addition to the unique capabilities of optoacoustic tomography to map other hemodynamic parameters such as blood volume and oxygen saturation in real time, thus is poised to provide new insights in neuroimaging and other applications.

Another important aspect to consider is the fact that the optoacoustic signal generated by droplets is proportional to the local light fluence, while the presence of a sparse distribution of particles barely affects the light fluence distribution. A cross-sectional image of the fluence in the brain can be obtained by superimposing images with droplets at different depths. A clear signal decay with depth is observed. The light fluence distribution could be estimated by fitting an exponentially decaying function to the measured signal intensities at different positions.

The feasibility to localize and track intravenously injected light absorbing microdroplets empowers localization optoacoustic tomography with otherwise unachievable capabilities for *in vivo* imaging of optically opaque tissues. Breaking through the acoustic diffraction barrier facilitates the volumetric visualization of microvascular structures not captured in standard optoacoustic tomography images. It is shown that localization optoacoustic tomography can achieve spatial resolution of ~20 $\mu$m across ~3 mm depth range through intact scalp and skull.

Apart from the high resolution provided, localization optoacoustic tomography enables locally quantifying the blood flow velocity with high accuracy due to the high temporal resolution (100 Hz), the short duration of the excitation pulse (<10 ns) and capability to reconstruct a 3D image with single shot excitation.

The signal analysis of a plurality of particles is explained by the following example:
The optoacoustic signal (initial pressure P0) at a given time point may be derived from the following equation:

$$PO = \Gamma\, \Phi\, \mu_a$$

Grueneisen parameter — $\Gamma$
Light fluence — $\Phi$
Absorption coefficient — $\mu_a$

$H = \Phi\mu_a$ Absorbed energy per unit volume

$H_{V,\Phi} = \mu_a V$ Absorbed energy in a given volume V per unit light fluence

The amplitude of the optoacoustic signal generated in a sub-resolution given volume V per unit light fluence is proportional to

$H_{V,\Phi}$

Optoacoustic signal at a given pixel of the image can be generated by determining the blood background. A typical resolution of optoacoustic tomography is ~150 $\mu$m, i.e., the voxel volume of a window derived from an image therefore might be $V_{voxel}$~150x150x150$\mu$m$^3$ = 3.37.10$^{-12}$m$^3$. Red blood cells have a volume $V_{RBC}$~90·10$^{-18}$m$^3$. These data can also be derived from an image preview.

Red blood cells represent approximately 40% of the blood volume. If an image voxel is filled with blood (inside a blood vessel), the number of red blood cells is

$$N_{RBC} = \frac{0.4 * V_{voxel}}{V_{RBC}} \sim 15000$$

Generally, the numbers of red blood cells included in a typical image voxel is less than this value.

For the optoacoustic signal from the injected particles the preferred criterion applies that for a particle to be detected at a given voxel, it has to generate the same (or higher) signal than the background signal generated by the RBCs, i.e.

Optoacoustic signal particle >= Optoacoustic signal 15000 RBCs

Proportional to $\mu_{a,p}V_p$     Proportional to $15000\mu_{a,RBC}V_{RBC}$

$$\mu_{a,p} \geq 15000\mu_{a,RBC}\frac{V_{RBC}}{V_p}$$

$$\left.\begin{array}{l} \mu_a = 2.3\varepsilon C \\ \varepsilon \text{ - Extinction coefficient} \\ C \text{ - Concentration} \end{array}\right\} \quad C_p \geq 15000\frac{\varepsilon_{Hb}}{\varepsilon_p}\frac{V_{RBC}}{V_p}C_{Hb} \longrightarrow \text{Haemoglobin}$$

In the following example we showcase the calculation steps: Excitation wavelength $\lambda$=800nm (typical in the near-infrared window)

$$\varepsilon_{Hb} \sim \frac{800cm^{-1}}{M}$$

$C_{Hb}$~5·10$^{-3}$M... in red blood cells

Considering

$\varepsilon_p$~200000$cm^{-1}$/M Particle containing a highly absorbing dye with extinction coefficient $\varepsilon_p$

For a particle with the same volume as a red blod cells - that can flow in blood

$$C_p \geq 300 \cdot 10^{-3} M$$

In an experiment performed, the following parameters have been applied

$$C_p = 200 \cdot 10^{-3} M$$

$\Gamma$ - 5 times higher than in water, which helps generating a strong signal

As mentioned above, the criterion employed is conservative, while generally the number is <15000 RBCs

Taken together, these unique enabling features can massively impact our understanding on the structural and functional properties of the brain microvasculature and other organs and regions in health and disease. Localization optoacoustic tomography may then permit identifying new key bio-markers of microcirculatory changes known to take place in clinically relevant conditions such as diabetes, cancer, cardiovascular disorders, or neurodegenerative diseases. It could additionally serve as an early diagnosis tool as well as provide new insights on disease progression and new capabilities to assess the efficacy of drugs and other treatments.

The strong absorbers employed break the continuity in the absorption distribution induced by densely-packed red blood cells and can be easily localized even if the angular coverage of the ultrasound sensor(s) is insufficient (<180°) for accurate optoacoustic reconstructions.

Thereby, localization optoacoustic tomography renders accurate vascular images under such limited-view conditions and overcomes a major limitation of optoacoustic tomography. Limited-view effects are associated to the speckle-free nature of optoacoustic tomography and have been avoided by artificially inducing speckle grains in the images, which is possible in localization optoacoustic tomography even if the injected particles cannot be individually distinguished.

Also important is the fact that the strength of the optoacoustic signal generated by an individual particle is proportional to the local light fluence, while the presence of sparsely distributed particles barely affects light attenuation. The average signals of droplets for different locations define an accurate estimator for light fluence distribution, a long-standing goal in biomedical optics. Particularly, optoacoustic image normalization with fluence is essential to achieve accurate readings of the concentration of agents that can serve as relevant disease bio-markers.

Fluence estimation may further be facilitated with monodisperse droplets, which can be synthetized with microfluidic chips. The unprecedented capabilities of localization optoacoustic tomography demonstrated in this work may potentially serve to foster the ongoing clinical translation of optoacoustics. The microdroplets employed in this work have a similar size to food and drug administration (FDA)-approved microbubbles used as ultrasound contrast agents.

On the other hand, FDA-approved indocyanine green (ICG) has a similar extinction coefficient as the IR780 dye used herein, and may serve as a localization optoacoustic tomography contrast agent if properly encapsulated in microparticles that successfully undergo the regulatory approval process. Overall, strongly absorbing microparticles smaller than red blood cells may then emerge as powerful blood pool optoacoustic contrast agents significantly outperforming existing dyes and nanoparticles that only increase the signal-to-noise ratio of the images to the detriment of the penetration depth. These new advantages, combined with the unique functional and molecular information provided by the large number of available optoacoustic targeted agents, can massively enhance the performance of optoacoustic tomography as a biomedical imaging tool.

In conclusion, the newly enabled capabilities for volumetrically mapping and characterizing microvascular structures *in vivo* with spatial resolution beyond the acoustic diffraction barrier and short acquisition times are poised to provide unprecedented insights on the anatomy and function of optically opaque complex organisms.

It is anticipated that the advantages provided by localization optoacoustic tomography, combined with the unique features of state-of-the-art optoacoustic tomography, will massively impact a large number of studies on clinically relevant diseases and further foster the growing use of optoacoustics as a biomedical imaging tool.

References

102      positioning - second step

102-2    preview - subsequent step

103      injection - third step

104      data acquisition - fourth step

105     data processing step - fifth step

**Claims**

1. Optoacoustic tomography method of a living organism, especially a human or an animal, **characterized by** the following steps;

> I Providing a living organism (101);
> II Positioning of an optoacoustic imaging system around the region of interest of the living organism (102)
> III Intravenous Injection (103) of an exogenous photoabsorbing agent as an emulsion containing
>
> > liquid and/or solid particles with an maximum diameter lower than 10 $\mu$m and
> > wherein the particles comprise at least a photoabsorbing substance;
>
> IV Acquisition (104) of at least one image after the injection of the photoabsorbing agent in step III;
> V Determination of data (105) derived from said image based on analyzing the signals of a plurality of said particles.

2. Optoacoustic tomography method according to claim 1, **characterized in that** the providing of a living organism as a human or animal in step I (101) comprises an anesthetization of said animal or human.

3. Optoacoustic tomography method according to claim 1 or 2, **characterized in that** the optoacoustic imaging system is provided with a laser, an ultrasonic transducer and a control and/or evaluation unit, wherein said optoacoustic imaging system is provided to generate an image with a single laser pulse.

4. Optoacoustic tomography method according to one of the preceding claims, **characterized in that** said method further comprises a reconstruction step (102-2) in step II that provides a real-time preview for the definition of the exact location for an injection of said photoabsorbing agent preferably before the injection.

5. Optoacoustic tomography method according to one of the preceding claims, **characterized in that** the acquisition of said at least one image is initiated within less than 10 Minutes, preferably within less than 60 Seconds after step III (103).

6. Optoacoustic tomography method according to one of the preceding claims, **characterized in that** the particles preferably comprise dichloromethane, polycaprolactone and/or silica particles, preferably at a concentration of at least 25 mass %.

7. Optoacoustic tomography method according to one of the preceding claims, **characterized in that** the absorbing substance comprise at least a dye, more preferably a cation heptamethine fluorescent dye, more preferably IR780 dye at a concentration of at least 50 mM and/or nanoparticles, preferably carbon nanotubes.

8. Optoacoustic tomography method according to one of the preceding claims, **characterized in that** the mass of the injected emulsion comprising the photoabsorbing agent is at most 10 g/kg weight of said animal or human, preferably between 1-5 g/kg.

9. Optoacoustic tomography method according to one of the preceding claims, **characterized in that** the method in step V (105) is based on localization of individual particles, preferably derived from a local point spread function of said particles.

10. Optoacoustic tomography method according to one of the preceding claims, **characterized in that** the laser pulse repetition rate is at least 1 Hz, preferably 50-150 Hz.

11. Optoacoustic tomography method according to one of the preceding claims, **characterized in that** the wavelength of said laser pulse is within a range between 650-1300 nm, more preferably between 680-900 nm.

12. Use of the method according to one of the preceding claims to generate 2D and/or 3D optoacoustic images and/or to measure the blood flow and/or the light fluence inside a tissue.

13. Optoacoustic 2D or 3D-Image, preferably derived by the method according to one of the preceding claims, wherein said image displays arterioles, venules and capillaries with a resolution of at least 50 $\mu$m, preferably between 5-25 $\mu$m.

14. Optoacoustic blood flow image for measuring velocities, preferably up to 100 mm/s.

15. Optoacoustic light fluence image of a tissue preferably as a function of excitation wavelength of a laser of an optoacoustic imaging system.

16. Photoabsorbing agent for the use in an optoacoustic method, especially according to one of the preceding claims, wherein said agent is an emulsion containing liquid and/or solid particles with an maximum diameter lower than 10 $\mu$m and wherein the particles comprise at least an absorbing substance.

Fig. 1

# Fig. 2

Europäisches
Patentamt

European
Patent Office

Office européen
des brevets

# EUROPEAN SEARCH REPORT

Application Number

EP 20 18 3260

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/306857 A1 (RAZANSKY DANIEL [DE] ET AL) 15 December 2011 (2011-12-15) * paragraphs [0017], [0026], [0033], [0063], [0065], [0068], [0071] - [0073], [0080]; figure 10 * | 1-12 | INV. A61B5/00 A61B5/0275 A61B5/0285 |
| X | US 2012/065490 A1 (ZHAROV VLADIMIR PAVLOVICH [US] ET AL) 15 March 2012 (2012-03-15) * paragraphs [0064], [0082], [0110], [0117] - [0123] * | 1-12 | |
| A | WO 2009/154963 A1 (UNIV TEXAS [US]; HOMAN KIMBERLY [US] ET AL.) 23 December 2009 (2009-12-23) * page 4, lines 4-5,16,33 * * page 5, line 9 * * page 6, lines 1,19 * * page 7, lines 15,16 * * page 11, line 14 - line 26 * * page 21, line 21 - line 22 * | 1-12 | |
| A | US 2013/190595 A1 (ORAEVSKY ALEXANDER A [US] ET AL) 25 July 2013 (2013-07-25) * paragraphs [0014], [0017], [0047], [0049], [0052], [0077]; figure 20B * | 1-12 | TECHNICAL FIELDS SEARCHED (IPC) A61B |

~~The present search report has been drawn up for all claims~~

| Place of search | Date of completion of the search | Examiner |
|---|---|---|
| The Hague | 24 November 2020 | Chau, Thoi Dai |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

                                         
& : member of the same patent family, corresponding
document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-12

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 20 18 3260

The Search Division considers that the present European patent application does not comply with the requirements of unity of invention and relates to several inventions or groups of inventions, namely:

1. claims: 1-12

    Optoacoustic tomography method of a living organism and use thereof
    ---

2. claim: 13

    Optoacoustic 2D or 3D-image
    ---

3. claim: 14

    Optoacoustic blood flow image for measuring velocities
    ---

4. claim: 15

    Optoacoustic light fluence image of a tissue
    ---

5. claim: 16

    Photoabsorbing agent
    ---

**EP 3 932 295 A1**

## ANNEX TO THE EUROPEAN SEARCH REPORT
## ON EUROPEAN PATENT APPLICATION NO.

EP 20 18 3260

This annex lists the patent family members relating to the patent documents cited in the above-mentioned European search report.
The members are as contained in the European Patent Office EDP file on
The European Patent Office is in no way liable for these particulars which are merely given for the purpose of information.

24-11-2020

| Patent document cited in search report | | Publication date | Patent family member(s) | | Publication date |
|---|---|---|---|---|---|
| US 2011306857 | A1 | 15-12-2011 | CA | 2731409 A1 | 28-01-2010 |
| | | | CN | 102137618 A | 27-07-2011 |
| | | | EP | 2344019 A1 | 20-07-2011 |
| | | | JP | 5749164 B2 | 15-07-2015 |
| | | | JP | 2011528923 A | 01-12-2011 |
| | | | US | 2011306857 A1 | 15-12-2011 |
| | | | WO | 2010009747 A1 | 28-01-2010 |
| US 2012065490 | A1 | 15-03-2012 | US | 2012065490 A1 | 15-03-2012 |
| | | | US | 2016354150 A1 | 08-12-2016 |
| WO 2009154963 | A1 | 23-12-2009 | NONE | | |
| US 2013190595 | A1 | 25-07-2013 | AU | 2013212213 A1 | 18-09-2014 |
| | | | CA | 2861979 A1 | 01-08-2013 |
| | | | EP | 2806803 A1 | 03-12-2014 |
| | | | JP | 6148257 B2 | 14-06-2017 |
| | | | JP | 2015507947 A | 16-03-2015 |
| | | | KR | 20140121451 A | 15-10-2014 |
| | | | US | 2013190595 A1 | 25-07-2013 |
| | | | WO | 2013112626 A1 | 01-08-2013 |

EPO FORM P0459

For more details about this annex : see Official Journal of the European Patent Office, No. 12/82

17

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **XU, M. ; WANG, L. V.** Universal back-projection algorithm for photoacoustic computed tomography. *Physical Review E,* 2005, vol. 71 (1), 016706